# EUROPEAN PATENT APPLICATION

(11) **EP 1 352 648 A1**
(43) Date of publication of application: **15.10.2003**
(21) Application number: 03007859.6
(22) Date of filing: 07.04.2003
(51) Int. Cl.: A61K 9/48

(54) **Pharmaceutical compositions of acetylsalicylic acid and omega-3 oils**

(30) Priority: 08.04.2002 IT MI20020731
(71) Applicant: Ibsa Institut Biochimique S.A., 6915 Lugano (CH)
(72) Inventor: Garavani, Alberto, 6900 Massagno (CH); Di Martino, Alessandro, 20123 Milan (IT); Marchiorri, Maurizio, 22039 Valbrona (IT); Bongiovanni, Giovanni, 20052 Monza (IT)
(74) Representative: Gervasi, Gemma, Dr.

(57) **Abstract**

The present invention provides pharmaceutical compositions of acetylsalicylic acid and omega-3 oils which enable safe and stable oral administration within the range of the narrow therapeutic index prescribed for the prevention of alterations of the cardiovascular system.

## Description

### Field of the invention

The present invention relates to pharmaceutical compositions of acetylsalicylic acid and omega-3 oils.

### Prior art

Acetylsalicylic acid (ASA) also known by the commercial name of aspirin, is a molecule used for more than a century as an anti-inflammatory, antiphlogistic, and analgesic in a classical therapeutic dose (500 mg) with release at gastric level and a virtually immediate action (about one hour between consumption and response). More recently as a platelet anti-aggregating agent in the prevention of thromboses with daily doses of 100 mg ("Aspirin Cardio") by delayed-release administration into the intestinal tract.

Oral administration of acetylsalicylic acid continues to present the serious problem of aggression and irritation of the gastric mucosa cells.

For long-duration therapeutic treatment different administration configurations must therefore be used.

### Summary of the invention

The applicant has unexpectedly and surprisingly discovered a pharmaceutical composition, suitable for oral administration, of acetylsalicylic acid in the form of soft elastic capsules; said composition enables administration able to develop the platelet anti-aggregation action. The conditions for creating a suspension in oil, and hence "liquid" or semi-liquid, have been found as an alternative to the normal solid formulations. Administering acetylsalicylic acid in a soft elastic capsule can be a more pleasant way for certain patients who prefer this pharmaceutical form for its characteristics of easy ingestion. As adjuvant in the platelet anti-aggregating activity an oil of the so-called "omega-3" group was used.

### Detailed description of the invention

One aspect of the present invention is therefore a pharmaceutical composition comprising:
1) acetylsalicylic acid in powder or crystal form
2) at least one oil of the so-called "omega-3" group
all enclosed in a soft elastic capsule, the casing or shell of which is based on gelatin or other components used for capsule manufacture.

The pharmaceutical composition of the present invention can present a coating of gastro-protective type.

In one embodiment of the present invention, in said composition at least one of the components chosen from acetylsalicylic acid and the soft elastic capsule casing is covered by a coating composed of a polymer insoluble in water or in the gastric environment, but enterosoluble, such as to enable controlled release of the acetylsalicylic acid. The polymer is chosen from the group consisting of:
hydroxypropyl-cellulose, hydroxypropylmethyl-cellulose, hydroxymethylethyl-cellulose and methyl-cellulose, cellulose derivatives in general, starch and derivatives, copolymers of methacrylic acid and its derivatives, polyvinylalcohol, polysaccharides and their derivatives, natural or synthetic waxes and lacquers, their derivatives and mixtures, ethyl-cellulose, aliphatic alcohols and their mixtures. Preferably the polymer insoluble in a gastric environment is chosen from the polymethacrylate group, and the water-insoluble polymer is ethyl-cellulose.

The coating process of the present invention is preferably:
a) microencapsulation by coacervation of the constituent granules of the acetylsalicylic acid powder or crystals,
b) film-coating the surface of each crystal by spray application of polymers in solution or emulsion or suspension,
c) film-coating the soft elastic capsule if the casing or shell thereof is to be coated.

In particular, ethyl-cellulose is used for microencapsulation by coacervation.

The gelatin, the basic constituent of the outer casing of the soft elastic capsules according to the present invention, is preferably a compound chosen from the group consisting of: type A gelatin, type B gelatin and their mixtures.

The omega-3 oil is chosen from the group consisting of lipids composed of glyceryl or ethyl esters of omega-3 C₁₈ -C₂₂ polyunsaturated fatty acids, the omega-3 oil preferably being chosen from the group consisting of mono-, di-, triglycerides or ethyl esters of C₂₀-C₂₂ polyunsaturated fatty acids or their mixtures, more preferably the omega-3 oil being chosen from the group consisting of triglycerides or ethyl esters of Docosahexaenoic acid (DHA), of Eicosapentaenoic acid (EPA), Docosapentaenoic acid (DPA) or their mixtures. Normally said omega-3 oils are extracted from fish oils (fish preferably chosen from the families of: Engraulidae, Carangidae, Clupeidae, Osmeridae, Salmonidae, Scombridae etc. Ref. Martindale 32^{nd} Ed., page 1276). Commercial oils include by way of example EPAX® 5500TG or LIPROMEGA® TG60. The omega-3 oil of the present invention can contain tocopherol as antioxidant.

The compositions of the present invention are in liquid or semi-liquid form, possibly together with further excipients, enclosed in soft elastic capsules.

The present invention enables the safe and reliable administration of acetylsalicylic acid and omega-3 oils within the range of the narrow therapeutic index prescribed for the prevention of alterations of the cardiovascular system and in particular is able to develop platelet anti-aggregation action.

The materials used for obtaining the capsules contemplated by the present invention are the usual gelatins (so-called type A or B) used in pharmaceutical practice.

The rigidity of the capsules contemplated by the present invention can be regulated, according to the type of capsule to be obtained, by using known pharmaceutically acceptable capsule plasticisers such as polyhydroxy alcohols, preferably glycerol, 1,2-propylene glycol, 1,2,6-hexanetriol, sorbitol, solutions of sorbitol and sorbitans (ANIDRISORB®), or other excipients suitable for the purpose.

Further usual optional components of the capsules contemplated by the present invention are water and preserving agents, always at the discretion of the pharmaceutical expert.

For the preferred embodiment of the present invention a soft elastic capsule is provided containing the composition comprising the acetylsalicylic acid as such or coated and the omega-3 oil, mixed with usual pharmaceutical excipients, as a purely indicative example: Soya oils, wheat germ oil, olive oil, medium chain triglycerides, coconut oil, palm oil, beeswax, GELEOL®, lecithin, GELUCIRE®, polyoxamers, polyethylene glycols, Aerosil, silica gel and all the excipients commonly used in pharmaceutical practice for manufacturing soft elastic capsules.

The present invention provides a soft elastic capsule consisting of a casing of gelatinous material, as such or coated, containing the acetylsalicylic acid, as such or coated and the omega-3 oil and possible excipients in a liquid or semi-liquid vehicle. In particular the soft elastic capsule contains an inner phase consisting of a semi-liquid, a paste, a suspension or dispersion comprising the various excipients and the acetylsalicylic acid as such or coated and the omega-3 oil.

The preferred manufacturing process for the aforesaid soft capsule comprises mixing the omega-3 oil, the acetylsalicylic acid as such or coated and possible excipients as a fluid vehicle. This latter is injected into a casing or shell of gelatin and plasticisers, possibly coated after manufacture, to give a finished soft capsule as in the classical process for manufacturing soft elastic capsules. In any event, any known process described in the pharmaceutical literature for obtaining soft elastic capsules with a semi-liquid content such as those described in "Remington's Pharmaceutical Sciences", 20^{th} edition, edited by Alfonso R. Gennaro, 2000, Lippincott Williams & Wilkins, ISBN 0-683-306472 is applicable for obtaining soft elastic capsules according to the present invention comprising acetylsalicylic acid as such or coated and omega-3 oil and possible excipients with a liquid or semi-liquid vehicle.

The preferred implementation of the present invention also has the further advantage of the relative ease with which unit doses perfectly homogeneous with each other are obtained.

In this respect, the known machines for producing soft elastic capsules with a semi-liquid content enable microdosing of the content (i.e. the inner phase) with a precision such as to limit the variation in the content from capsule to capsule to within the limits of the pharmacopoeia.

The excipients usable together with the liquid vehicles include all the usual pharmaceutically acceptable solid additives which can be used, dispersed or dissolved, to modify the viscosity of the capsule content or the release characteristics of the omega-3 oil and of the aspirin as such or coated by the vehicle. The liquid or semi-liquid vehicles include by way of example glycerol, ethanol, polyethylene glycol (particularly with molecular weight 200-6000), glycofurol (tetrahydrofurfuryl alcohol polyethylene glycol ether; Sigma T3396), 1,2-propylene glycol, pharmaceutically acceptable oils, or non-ionic surfactants, for example polysorbates (polysorbate 20 or 80), or various Tweens® (i.e.monolaurates, monooleates, monopalmitates, monostearates, trioleates or tristearates of polyoxyethylenesorbitan, for example Tween 80, silica gel, Aerosil) or other vehicles (or their mixtures) commonly used in the pharmaceutical industry for the formulation of soft elastic capsules with a semi-liquid content.

A preferred example of the capsule material is gelatin (either type A obtained from pig skin by acid treatment or type B obtained from animal bone and skin by alkaline treatment), whereas the plasticisers usable for regulating the capsule elasticity can be glycerol, 1,2-propylene glycol, 85% sorbitol/sorbitan solution etc. As known in the pharmaceutical industry, the gelatinous material of the capsule and the semi-liquid content of the capsule must be compatible and hence, for the capsule material, the use of plasticisers which can be present (even at different percentages) in the liquid or semi-liquid vehicle is preferred, for example glycerol.

The pharmaceutical compositions of the present invention are suitable for use in the prevention of cardiovascular diseases with the purpose of anti-thrombotic prophylaxis.

Some illustrative but non limitative examples are given hereinafter of the compositions according to the present invention.

### EXPERIMENTAL PART

### Example 1

Soft elastic capsules indicated respectively by SEC 1 and SEC 2 are formed comprising as active principle acetylsalicylic acid (ASA) as such and acetylsalicylic acid microencapsulated in ethy-cellulose respectively. The shell or casing of the capsule SEC 1 and SEC 2 is composed of gelatin 150 bloom, ANIDROSORB® and water which evaporates during the drying stage.

The compositions of the two capsules, expressed in mg per capsule, refer to the capsule content and are shown in Table 1.

**TABLE 1**

| **COMPONENTS** | **SEC 1** mg/capsule | **SEC 2** mg/capsule |
|---|---|---|
| EPAX ® 5500 TG | 204.510 | 204.510 |
| Soya lecithin | 8.200 | 8.200 |
| Beeswax | 40.100 | 40.100 |
| Palm oil | 24.600 | 24.600 |
| Coconut oil | 24.600 | 24.600 |
| ASA | 100.250 | 103.093 |

The stability of the capsules SEC 1 and SEC 2 was verified by controlling the quantity expressed in percentage of acetylsalicylic acid with respect to the theoretical after maintaining the two capsules for 150 days in a blister pack of polyvinylchloride/polyvinylidenechloride/aluminium (PVC/PVDC/ALU) and aluminium/aluminium (ALU/ALU).

### Example 2 (comparison)

A soft elastic capsule indicated by SEC 3 was formed comprising as active principle acetylsalicylic acid (ASA) but which does not contain omega-3 oil. The shell or casing of the capsule SEC 3 is identical to the casing of the capsules SEC 1 and SEC 2.

The composition of the capsule, expressed in mg per capsule, refers to the capsule content and is given in Table 2.

**TABLE 2**

| **COMPONENTS** | **SEC 3** mg/capsule |
|---|---|
| Polyethylene glycol 400 | 300.000 |
| ASA | 100.250 |

On verifying the stability of the capsule SEC 3 an acetylsalicylic acid content was found which was less in a shorter time than the acetylsalicylic acid content found after 150 days for the capsules SEC 1 and SEC 2, a structural deterioration also being observed for the capsule SEC 3.

## Claims

1. Pharmaceutical composition comprising:
1) acetylsalicylic acid in powder or crystal form,
2) at least one oil of the so-called "omega-3" group. all enclosed in a soft elastic capsule.

2. Composition as claimed in claim 1 wherein at least one of the components chosen from acetylsalicylic acid and the casing or shell of the soft elastic capsule is covered by a coating composed of a polymer insoluble in water or in a gastric environment but enterosoluble, such as to enable controlled release of the acetylsalicylic acid.

3. Composition as claimed in claim 2 wherein the constituent polymer of the coating is chosen from the group consisting of: hydroxypropyl-cellulose, hydroxypropylmethyl-cellulose, hydroxymethylethyl-cellulose and methyl-cellulose, cellulose derivatives in general, starch and derivatives, copolymers of methacrylic acid and its derivatives, polyvinylalcohol, polysaccharides and their derivatives, natural or synthetic waxes and lacquers, their derivatives and mixtures, ethyl-cellulose, aliphatic alcohols and their mixtures.

4. Composition as claimed in claim 3 wherein the polymer insoluble in a gastric environment is chosen from the polymethacrylate group and the water-insoluble polymer is ethyl-cellulose.

5. Composition as claimed in claim 1 wherein the basic constituent of the outer casing of the soft elastic capsule is gelatin chosen from the group consisting of: type A gelatin, type B gelatin or their mixtures.

6. Compositions as claimed in claim 1 wherein the plasticiser of the outer casing or shell of the soft elastic capsule is chosen from the group consisting of polyhydroxy alcohols, preferably glycerol, 1,2-propylene glycol, 1,2,6-hexanetriol, sorbitol, solutions of sorbitol and sorbitans (ANIDRISORB®).

7. Compositions as claimed in claim 1 wherein the omega-3 oil is chosen from the group consisting of lipids composed of glyceryl or ethyl esters of omega-3 C₁₈-C₂₂ polyunsaturated fatty acids.

8. Compositions as claimed in claim 7 wherein the omega-3 oil is chosen from the group consisting of mono-, di-, triglycerides or ethyl esters of C₂₀-C₂₂ polyunsaturated fatty acids or their mixtures.

9. Composition as claimed in claim 8 wherein the omega-3 oil is chosen from the group consisting of triglycerides or ethyl esters of Docosahexaenoic acid (DHA), of Eicosapentaenoic acid (EPA), of Docosapentaenoic acid (DPA) or their mixtures.
